(19) Europäisches Patentamt
European Patent Office
Office européen des brevets

(11) Veröffentlichungsnummer: **0 073 970**
**B1**

(12) # EUROPÄISCHE PATENTSCHRIFT

(45) Veröffentlichungstag der Patentschrift: **02.03.88**

(51) Int. Cl.$^4$: **C 07 D 253/06,** C 07 D 405/04

(21) Anmeldenummer: **82107480.4**

(22) Anmeldetag: **17.08.82**

(54) **Verfahren zur Herstellung von 4-Methyl-5-oxo-3-thioxotetrahydro-1,2,4-(2H, 4H)-triazinen.**

(30) Priorität: **29.08.81 DE 3134230**

(43) Veröffentlichungstag der Anmeldung:
**16.03.83 Patentblatt 83/11**

(45) Bekanntmachung des Hinweises auf die Patenterteilung:
**02.03.88 Patentblatt 88/09**

(84) Benannte Vertragsstaaten:
**AT BE CH DE FR GB IT LI NL**

(56) Entgegenhaltungen:
**EP-A-0 015 452**
**EP-A-0 035 706**
**EP-A-0 035 708**
**DE-A-1 670 912**
**DE-A-2 708 185**
**DE-A-2 908 963**

**Die Akte enthält technische Angaben, die nach dem Eingang der Anmeldung eingereicht wurden und die nicht in dieser Patentschrift enthalten sind.**

(73) Patentinhaber: **BAYER AG**
**Konzernverwaltung RP Patentabteilung**
**D-5090 Leverkusen 1 Bayerwerk (DE)**

(72) Erfinder: **Bonse, Gerhard, Dr.**
**Wolfskaul 3**
**D-5000 Köln 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 073 970 B1

**Beschreibung**

Die vorliegende Erfindung betrifft ein neuartiges, von Acylcyaniden ausgehendes Verfahren zur Herstellung von weitgehend bekannten 6-substituierten 4-Methyl-5-oxo-3-thioxotetrahydro-1,2,4-(2H, 4H)-triazinen (I), welche also Zwischenprodukte zur Synthese von bekannten herbizid wirksamen 1,2,4-Triazin-5(4H)-onen verwendet werden können (vergleiche z.B. DE—OS 16 70 912, DE—OS 29 08 963, DE—OS 29 08 964, DE—OS 29 38 384 und DE—OS 30 37 300).

Zur Herstellung der Triazine (I) ist eine Reihe von Verfahren bekannt geworden.

Ausgehend von α-Ketocarbonsäuren bzw. deren Salzen können die Triazine (I) durch mehrstündiges Kochen, bis zu lo Stunden, in Wasser mit 4-Methyl-thiosemicarbazid in Ausbeuten von 80% der Theorie hergestellt werden (vergleiche "Fortschritte der chemischen Forschung" Bd. 5, S. 189 (1965); DE—OS 16 70 912; DE—OS 29 38 384).

Nach DE—OS 29 08 964 können die Triazine (I) weiterhin durch Kondensation von 2-Mercapto-2-cyclohexylidenessigsäure mit 4-Methyl-thio-semicarbazid hergestellt werden. 2-Mercapto-2-cyclohexylidenessigsäure wird nach alkalischer Hydrolyse des Kondensationsproduktes Cyclohexyliden-N-methylrhodanin aus Cyclohexanon und N- Methylrhodanin erhalten.

Die genannten vielstufigen, vorbekannten Verfahren haben den Nachteil, daß viele der als Ausgangstoffe benötigten α-Ketocarbonsäuren nur schwer zugänglich sind. Sie müssen zum Teil nach technisch außerordentlich aufwendigen Methoden in mehreren Stufen mit zum Teil nur unbefriedigenden Ausbeuten hergestellt werden (vergleiche die zusammenfassende Darstellung: Synthesewege zu α-Ketocarbonsäuren in "Comprehensive Organic Chemistry" Bd. 2,779 (1979)).

Aus DE—A1—2 708 185 ist ferner ein Verfahren bekannt, wonach bestimmte aliphatische und cycloaliphatische Acylcyanide direkt zu den entsprechenden α-Ketocarbonsäureamiden bzw. den freien α-Ketocarbonsäuren hydrolysiert werden können.

Es wurde nun gefunden, daß man 4-Methyl-5-oxo-3-thioxotetrahydro-1,2,4-(2H, 4H)-triazine (I), welche in den beiden tautomeren Formen (Ia) und (Ib) vorliegen können,

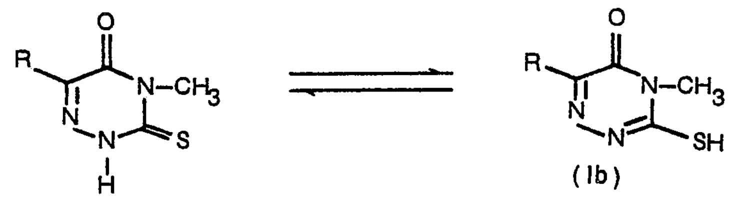

(Ia) (Ib) (I)

worin

R für einen gegebenenfalls durch Alkoxy mit 1 bis 4 Kohlenstoffatomen, Carbalkoxy mit 1 bis 4 Kohlenstoffatomen in der Alkoxygruppe, Nitro, Cyano und/oder Halogen substituierten, geradkettigen oder verzweigten Alkylrest mit 1 bis 4 Kohlenstoffatomen; für einen gegebenenfalls durch Alkyl, Alkoxy oder Carbalkoxy mit jeweils bis zu 4 Kohlenstoffatomen, Nitro, Cyano und/oder Halogen substituierten Cycloalkylrest mit 3 bis 6 Kohlenstoffatomen im Ringsystem; für einen gegebenenfalls durch Alkyl, Alkoxy oder Carbalkoxy mit jeweils bis zu 4 Kohlenstoffatomen, Nitro und/oder Halogen substituierten Phenyl- oder Naphthylrest; oder für einen gegebenenfalls durch Alkyl, Alkoxy oder Carbalkoxy mit jeweils bis zu 4 Kohlenstoffatomen, Nitro, Cyano und/oder Halogen substituierten 5- oder 6-gliedrigen heterocyclischen Rest steht, der 1 bis 3 Heteroatome wie Sauerstoff, Schwefel und/oder Stickstoff im Ring enthalten und außerdem mit einem Benzolring annelliert sein kann,

ausgehend von Acylcyaniden in überraschend einfacher Weise mit hoher Ausbeute und in hoher Reinheit erhält, wenn man Acylcyanide der allgemeinen Formel II

$$R—CO—CN \qquad (II)$$

in welcher

R die oben angegebene Bedeutung hat,

mit einem Carbonsäureanhydrid der allgemeinen Formel III

$$R^1—CO—O—CO—R^1 \qquad (III)$$

in welcher

$R^1$ für einen gegebenenfalls chlorsubstituierten Alkylrest mit 1 bis 4 Kohlenstoffatomen oder für einen Phenylrest steht,

in Gegenwart einer starken Säure, ausgewählt aus der Gruppe bestehend aus anorganischen Säuren, Lewis-Säuren, aliphatischen oder aromatischen Sulfon- und Phosphonsäuren sowie

Halogenalkancarbonsäuren, und gegebenenfalls in Gegenwart eines Lösungsmittels bei Temperaturen zwischen −50 und +150°C umsetzt und das so erhaltene Reaktionsgemisch anschließend direkt mit 4-Methyl-thiosemicarbazid ($CH_3$—NH—CS—NH—$NH_2$) umsetzt.

Die so erhaltenen Triazine der Formel (I) können nachfolgend nach bekannten Methoden in die herbizid wirksammen 6-Alkyl(Aryl)-3-dimethylamino-4-methyl-1,2,4-triazin-5-(4H)-one überführt werden (vergleiche DE—OS 16 70 912; DE—OS 29 08 963; DE—OS 29 08 964; DE—OS 29 38 384).

Das erfindungsgemäße Verfahren beinhaltet eine gegenüber dem Stand der Technik absolut neuartige und vorteilhafte Verfahrensweise, wobei es in überraschend glattem und einheitlichem Reaktionsverlauf erstmalig gelingt, Acylcyanide unter milden Bedingungen in einem 'Eintopfverfahren', ohne daß irgendwelche Zwischenprodukte isoliert werden müssen, direkt mit nahezu quantitativer Ausbeute in außerordentlich reine 4-Methyl-5-oxo-3-thioxo-tetrahydro-1,2,4-(2H, 4H)-triazine (I) zu überführen.

Das erfindungsgemäße Verfahren vermeidet die zuvor erwähnten, mit den vergleichbaren vorbekannten Verfahren verbundenen Nachteile; dies bedeutet eine sehr erhebliche technische Vereinfachung.

Verwendet man als Acylcyanid der allgemeinen Formel (II) Pivaloylcyanid sowie Essigsäureanhydrid als Carbonsäureanhydrid der allgemeinen Formel (III) und konzentrierte Schwefelsäure als starke Säure, so kann der Reaktionsverlauf nach dem erfindungsgemäßen Verfahren zusammenfassend nochmals durch das folgende Formelschema dargestellt werden:

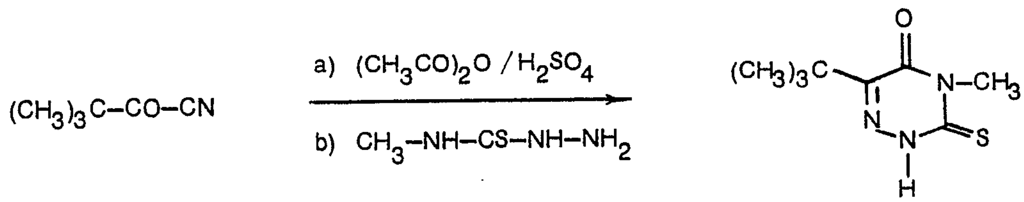


Die also Ausgangstoffe einzusetzenden Acylcyanide sind durch Formel (II) allgemein definiert. In dieser Formel steht R für geradkettiges oder verzweigtes Alkyl mit 1 bis 4 Kohlenstoffatomen, wobei jeder dieser Alkylreste substituiert sein kann durch Alkoxy mit 1 bis 4 Kohlenstoffatomen, Carbalkoxy mit 1 bis 4 Kohlenstoffatomen in der Alkoxygruppe, Nitro, Cyano und/oder Halogen (wie zum Beispiel Fluor, Chlor, Brom oder lod); ferner steht R für gegebenenfalls durch Alkyl, Alkoxy oder Carbalkoxy mit jeweils bis zu 4 Kohlenstoffatomen, Nitro, Cyano und/oder Halogen (wie z.B. Fluor, Chlor und Brom), substituiertes Cycloalkyl mit 3 bis 6 Kohlenstoffatomen im Ringsystem. Weiterhin steht R für gegebenenfalls durch Alkyl, Alkoxy oder Carbalkoxy mit jeweils bis zu 4 Kohlenstoffatomen, Nitro und/oder Halogen (wie zum Beispiel Fluor, Chlor und Brom), substituiertes Phenyl oder Naphthyl; schließlich steht R für gegebenenfalls durch Alkyl, Alkoxy oder Carbalkoxy mit jeweils bis zu 4 Kohlenstoffatomen, Nitro, Cyano und/oder Halogen (wie z.B. Fluor, Chlor und Brom), substituierte 5- oder 6-gliedrige heterocyclische Reste, die 1 bis 3 Heteroatome wie Sauerstoff, Schwefel und/oder Stickstoff im Ring enthalten können und außerdem mit einem Benzolring annelliert sein können. Als Beispiele für insbesondere in Frage kommende heterocyclische Reste seien genannt: Morpholinyl, Imidazolyl, Pyrazolyl, Pyrrolyl, Isoxazolyl, Piperidinyl, Oxazolyl, 1,2,4-Triazol-1-yl, 1,2,4-Triazol-4-yl, 1,2,3-Triazolyl, 1,2,4-thiadiazol-2-yl, Benzimidazolyl und Furanyl.

Die Acylcyanide der Formel (II) sind zum Teil bekannt; noch nicht bekannte Acylcyanide können nach bekannten Verfahren einfach hergestellt werden (vergleiche Angew. Chem. *68*, S. 425—435 (1956); ferner DE—OS'en 26 14 240, 26 14 241, 26 14 242, 27 08 182, 27 08 183, DE—PS 26 60 344).

Als im Rahmen dieser Erfindung besonders bevorzugtes Acylcyanid ist das Pivaloylcyanid zu nennen.

Die weiterhin als Ausgangsstoffe einzusetzenden Carbonsäureanhydride sind durch Formel (III) allgemein definiert. In dieser Formel steht $R^1$ für gegebenenfalls chlorsubstituiertes Alkyl mit 1 bis 4 Kohlenstoffatomen oder für Phenyl.

Die Carbonsäureanhydride der Formel (III) sind zum Teil großtechnisch verfügbar bzw. nach allgemein bekannten Methoden z.B. aus den entsprechenden Carbonsäuren herstellbar.

Im Rahmen dieser Erfindung besonders bevorzugte Carbonsäureanhydride sind Essigsäureanhydrid, Propionsäureanhydrid und die Anhydride der Chloressigsäuren.

Die erfindungsgemäße Umsetzung wird in Gegenwart einer starken Säure durchgeführt, welche ausgewählt wird aus der Gruppe bestehend aus anorganischen Säuren, (wie konzentrierte Schwefelsäure, Halogenwasserstoffsäuren, z.B. wasserfreier Chlorwasserstoff und Bromwasserstoff, sowie Salpetersäure, Perchlorsäure und Phosphorsäure), Lewis-Säuren (wie Bortrifluorid, Aluminiumchlorid oder Zinkchlorid), alipatischen oder aromatischen Sulfon- und Phosphonsäuren sowie Halogenalkancarbonsäure (wie z.B. trichloressigsäure). Bevorzugt werden Sauerstoffsäuren; insbesondere wird konzentrierte Schwefelsäure verwendet.

Es ist möglich, die erfindungsgemäße Umsetzung in Gegenwart einer oder mehrerer solcher Säuren durchzuführen.

Die Reaktionstemperaturen können in einem größeren Bereich variiert werden. Im allgemeinen arbeitet man, wie oben angegeben, bei Temperaturen zwischen etwa −50 und +150°C, vorzugsweise zwischen etwa 0 und 100°C, insbesondere zwischen 50 und 90°C.

Die Reaktion wird im allgemeinen bei Normaldruck durchgeführt.

Die Umsetzung kann in Abwesenheit oder in Gegenwart eines Lösungsmittels bzw. Lösungsvermittlers durchgeführt werden. Als Lösungsvermittler kommen bestimmte organische Lösungsmittel in Frage; besonders geeignet sind Eisessig und Dichlormethan, ferner Dialkylether, wie Diethyl- oder Diisopropylether, und Diarylether, wie z.B.Diphenylether.

Bei der Durchführung des erfindungsgemäßen Verfahrens setzt man im allgemeinen auf 1 Mol Acylcyanid der Formel (II) 0,5 bis 6 Mol, vorzugsweise 0,8 bis 4 Mol Carbonsäureanhydrid der Formel (III) ein; besonders bevorzugt ist ein Molverhältnis Acylcyanid (II) zu Carbonsäureanhydrid (III) von 1:1 bis 1:2.

Die für die Durchführung des erfindungsgemäßen Verfahrens erforderlichen Säuren werden in katalytischen bis überstöchiometrischen Mengen eingesetzt. Im allgemeinen setzt man auf 1 Mol Acylcyanid (II) 0,5 bis 10 Mol, vorzugsweise, 0,8 bis 8 Mol, besonders bevorzugt 1 bis 4 Mol Säure ein.

Besonders vorteilhaft ist ein Molverhältnis Carbonsäureanhydrid (III) zu der starken Säure von 1:2.

Ferner ist es besonders zweckmäßig, Acylcyanid (II) und 4-Methylthiosemicarbazid in äquimolaren Mengen einzusetzen.

Damit ergibt sich, daß es bei der Durchführung des erfindungsgemäßen Verfahrens besonders günstig ist, Acylcyanid (II), Carbonsäureanhydrid (III), die starke Säure und 4-Methyl-thiosemicarbazid im Molverhältnis von 1:1:2:1 bis 1:2:4:1 umzusetzen.

Wenn das Verfahren in technischem Maßstab durchgeführt wird, kann es jedoch vorteilhaft sein, unter Konstanthalten des Molverhältnisses Carbonsäureanhydrid (III) zur starken Säure von 1:2 einen Ueberschuß dieser beiden Komponenten einzusetzen, um das anfallende Reaktionsgemisch gut rührbar zu halten.

Zweckmäßigerweise geht man bei der Durchführung des erfindungsgemäßen Verfahrens wie folgt vor:

Man legt die starke Säure bzw. ein Gemisch aus Lösungsmittel und der starken Säure vor und fügt nacheinander das Carbonsäureanhydrid (III) und das Acylcyanid (II) zu; das so erhaltene Reaktionsgemisch wird entweder sofort nach beendeter Zugabe des Acylcyanids oder nach einer gewissen Nachrührzeit (von max. 3 Stunden) in eine vorzugsweise mineralsaure wäßrige oder wäßrig-alkoholische Lösung oder in eine wäßrige Suspension von 4-Methyl-thiosemicarbazid eingetragen. Es ist jedoch auch möglich, umgekehrt die 4-Methyl-thiosemicarbazid-Lösung bzw. -Suspension in das vorerwähnte Reaktionsgemisch einzutragen.

Die so erhaltene Reaktionsmischung wird anschließend durch Zusatz von Basen annähernd schwach sauer bis neutral gestellt und erwärmt.

Als Basen können im Prinzip alle üblicherweise als Säurebindemittel verwendbaren anorganischen und organischen basischen Verbindungen eingesetzt werden. Hierzu gehören vorzugsweise Alkali- und Erdalkali-metalloxide, -hydroxide, -carbonate und -hydrogencarbonate, wobei als Metalle vorzugsweise in Frage kommen: Natrium, Kalium und Calcium; sowie tertiäre Amine, wie Trimethylamin, Triethylamin, Pyridin, Lauryldimethylamin, Stearyldimethylamin, N,N-Diethylcyclohexylamin, N-Ethylpiperidin, N-Methylpyrrolidin, α, β- und γ-Picolin, N-Propylpiperidin, Chinolin, Isochinolin, Chinoxalin, Tri-n-amylamin, Tri-n-propylamin oder N,N-Dimethylbenzylamin.

Die Reaktionszeiten betragen im allgemeinen 1 bis 3 Stunden. Das Reaktionsprodukt fällt in der Regel in kristalliner Form aus und kann in üblicher Weise durch Filtration oder durch Extraktion isoliert werden.

Hierzu geeignete Extraktionsmittel sind mit Wasser nicht beliebig mischbare Lösungsmittel, beispielsweise Ether wie Diethylether oder Diisopropylether, Ester wie z.B. Essigsäureethylester, Ketone wie z.B. Methylisobutylketon, Halogenkohlenwasserstoff wie z.B. Dichlormethan, Chlorbenzol oder Dichlorbenzol, ferner Aromaten wie z.B. Benzol, Toluol, o-Xylol, Ethylbenzol, Cumol oder Nitrobenzol. Vorzugsweise verwendet man Dichlormethan.

Die nach dem erfindungsgemäßen Verfahren erhaltenen Triazine (I) können in bekannter Weise durch Umsetzung mit einem niederen Alkylhalogenid, wie beispielsweise Methylbromid, in Gegenwart einer Base, wie Natriumhydroxid, in wäßriger Lösung bei Temperaturen zwischen 0 und 50°C zu 3-Alkylthiotriazinonen der Formel (IV) alkyliert werden. Daraus werden dann durch Umsetzung mit Dimethylamin in Gegenwart von aliphatischen Carbonsäuren und einer organischen Sulfosäure die bekanntermaßen herbizid wirksamen 6-Alkyl(Aryl)-3-dimethyl-amino-4-methyl-1,2,4-triazin-5-(4H)-one (V) erhalten (vergleiche DE—OS 16 70 912, 29 08 963, 29 08 964 und DE—OS 29 38 384).

Dieser Reaktionsablauf sei durch das folgende Formelscheme noch einmal illustriert:

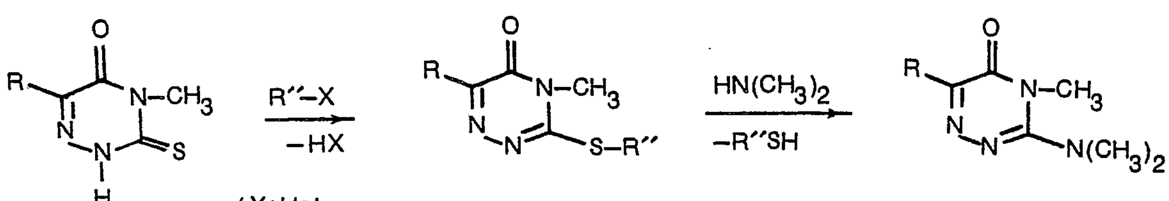


(Ia) (X: Halogen R'': Nieder= alkyl) (IV) (V)

Die nachfolgenden Beispiele sollen die Erfindung näher erläutern, aber nicht einschränken.

## Herstellungsbeispiele

Beispiel 1 6-ter.-Butyl-4-methyl-5-oxo-3-thioxo-tetrahydro-1,2,4-(2H, 4H)triazin (1)

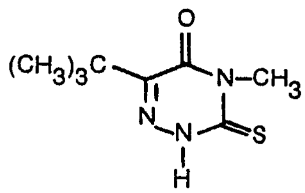

(I)

In 98,0 g (1,0 Mol) vorgelegte konzentrierte Schwefelsäure werden jeweils bei Raumtemperatur zunächst 51,2 g (0,5 Mol) Essigsäureanhydrid und anschließend 27,8 g (0,25 Mol) Pivaloylcyanid eingetragen. Nach einstründigem Nachrühren bei 40°C wird dieses Reaktionsgemisch in eine Lösung von 23,3 g (0,25 Mol) 4-Methyl-thiosemicarbazid in 200 ml 1n — HCl eingerührt. Nach beendeter Zugabe wird eine weitere Stunde bei Raumtemperatur nachgerührt. Anschließend wird mit 1200 ml 2n NaOH ein pH-Wert von ca. 5 eingestellt und eine Stunde auf 80°C erwärmt. Nach dem Abkühlen wird das ausgefallene Reaktionsprodukt abgesaugt, mit 200 ml Wasser gewaschen und getrocknet. Man erhält 45,8 g (92 % der Theorie) der Verbindung (1) als farblose Kristalle vom Schmelzpunkt 214°C; Gehalt nach gaschromatographischer Bestimmung >99%. Für anschließende Umsetzungen sind keine weiteren Reinigungsoperationen erforderlich.

In entsprechender Weise können die folgenden Verbindungen der allgemeinen Formel I

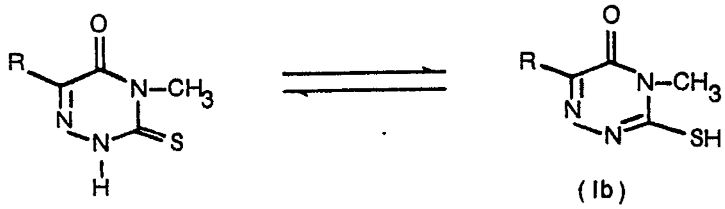

(Ia) (Ib) (I)

hergestellt werden:

TABELLE 1

| Beispiel Nr. | R | Schmelzpunkt(°C) |
|---|---|---|
| 2 | $-CH_3$ | 186 |
| 3 | $-CH(CH_3)_2$ | 191 |
| 4 | $-CH_2CH(CH_3)_2$ | 135 |
| 5 | $-CH(CH_3)C_2H_5$ | 117 |
| 6 | $-CH_2C(CH_3)_3$ | 171 |
| 7 | 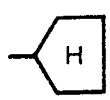 | 194 |
| 8 | 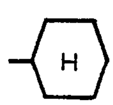 | 189 |
| 9 | 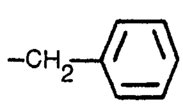 | 184 |
| 10 | 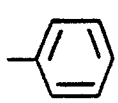 | 223 |

TABELLE 1 (Fortsetzung)

| Beispiel Nr. | R | Schmelzpunkt(°C) |
|---|---|---|
| 11 | 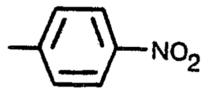 | 260 |
| 12 | 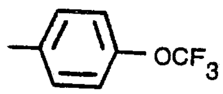 | 198 |
| 13 | 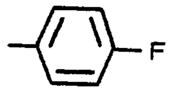 | 217 |
| 14 | 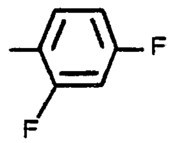 | 210 |
| 15 | 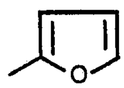 | 247 |

**Patentansprüche**

1. Verfahren zur Herstellung von 4-Methyl-5-oxo-3-thioxotetrahydro-1,2,4-(2H, 4H)-triazinen (I), welche in den beiden tautomeren Formen (Ia) und (Ib) vorleigen können,

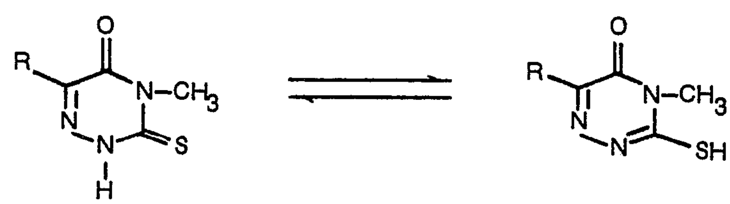

(I)

(Ia) (Ib)

worin

R für einen gegebenenfalls durch Alkoxy mit 1 bis 4 Kohlenstoffatomen, Carbalkoxy mit 1 bis 4 Kohlenstoffatomen in der Alkoxygruppe, Nitro, Cyano und/oder Halogen substituierten, geradkettigen oder verzweigten Alkylrest mit 1 bis 4 Kohlenstoffatomen; für einen gegebenenfalls durch Alkyl, Alkoxy oder Carbalkoxy mit jeweils bis zu 4 Kohlenstoffatomen, Nitro, Cyano und/oder Halogen substituierten Cycloalkylrest mit 3 bis 6 Kohlenstoffatomen im Ringsystem; für einen gegebenenfalls durch Alkyl, Alkoxy oder Carbalkoxy mit jeweils bis zu 4 Kohlenstoffatomen, Nitro und/oder Halogen substituierten Phenyl- oder Naphthylrest; oder für einen gegebenenfalls durch Alkyl, Alkoxy oder Carbalkoxy mit jeweils bis zu 4 Kohlenstoffatomen, Nitro, Cyano und/oder Halogen substituierten 5- oder 6-gliedrigen heterocyclischen Rest steht, der 1 bis 3 Heteroatome wie Sauerstoff, Schwefel und/oder Stickstoff im Ring enthalten und außerdem mit einem Benzolring annelliert sein kann,

dadurch gekennzeichnet, daß man Acylcyanide der allgemeinen Formel II

$$R—CO—CN \qquad (II)$$

in welcher

R die oben angegebene Bedeutung hat,

mit einem Carbonsäureanhydrid der allgemeinen Formel III

$$R^1—CO—O—CO—R^1 \qquad (III)$$

in welcher

$R^1$ für einen gegebenenfalls chlorsubstituierten Alkylrest mit 1 bis 4 Kohlenstoffatomen oder für einen Phenylrest steht,

in Gegenwart einer starken Säure, ausgewählt aus der Gruppe bestehend aus anorganischen Säuren, Lewis-Säuren, aliphatischen oder aromatischen Sulfon- und Phosphonsäuren sowie Halogenalkancarbonsäuren, und gegebenenfalls in Gegenwart eines Lösungsmittels bei Temperaturen zwischen −50 und +150°C umsetzt und das so erhaltene Reaktionsgemisch anschließend direkt mit 4-Methyl-thiosemicarbazid ($CH_3$—NH—CS—NH—$NH_2$) umsetzt.

2. Verfahren nach Anspruch 1, dadurch gekennzeichnet, daß man die Umstzung bei Temperaturen zwischen 0 und 100°C durchführt.

3. Verfahren nach Anspruch 1, dadurch gekennzeichnet, daß man Acylcyanid (II) und Carbonsäureanhydrid (III) im Molverhältnis von 1:0,5—6 einsetzt.

4. Verfahren nach Anspruch 3, dadurch gekennzeichnet, daß man Acylcyanid (II) und Carbonsäureanhydrid (III) im Molverhältnis von 1:0,8—4 einsetzt.

5. Verfahren nach Anspruch 4, dadurch gekennzeichnet, daß man Acylcyanid (II) und Carbonsäureanhydrid (III) im Molverhältnis von 1:1—2 einsetzt.

6. Verfahren nach Anspruch 1, dadurch gekennzeichnet, daß man Acylcyanid (II) und starke Säure im Molverhältnis von 1:0,5—10 einsetzt.

7. Verfahren nach Anspruch 6, dadurch gekennzeichnet, daß man Acylcyanid (II) und starke Säure im Molverhältnis von 1:0,8—8 einsetzt.

8. Verfahren nach Anspruch 7, dadurch gekennzeichnet, daß man Acylcyanid (II) und starke Säure im Molverhältnis von 1:1—4 einsetzt.

9. Verfahren nach Anspruch 1, dadurch gekennzeichnet, daß man Carbonsäureanhydrid (III) und starke Säure im Molverhältnis 1:2 einsetzt.

10. Verfahren nach Anspruch 1, dadurch gekennzeichnet, daß man Acylcyanid (II), Carbonsäureanhydrid (III), starke Säure und 4-Methyl-thiosemicarbazid im Molverhältnis von 1:1:2:1 bis 1:2:4:1 einsetzt.

11. Verfahren nach Anspruch 1, dadurch gekennzeichnet, daß man als Acylcyanid der Formel (II) Pivaloylcyanid einsetzt.

12. Verfahren nach Anspruch 1, dadurch gekennzeichnet, daß man als Carbonsäureanhydrid der Formel (III) Essigsäureanhydrid einsetzt.

13. Verfahren nach Anspruch 1, dadurch gekennzeichnet, daß man als starke Säure konzentrierte Schwefelsäure einsetzt.

## Revendications

1. Procédé de production de 4-méthyl-5-oxo-3-thioxotétrahydro-1,2,4-(2H, 4H)-triazines (I), qui peuvent se présenter sous deux formes tautomères (Ia) et (Ib)

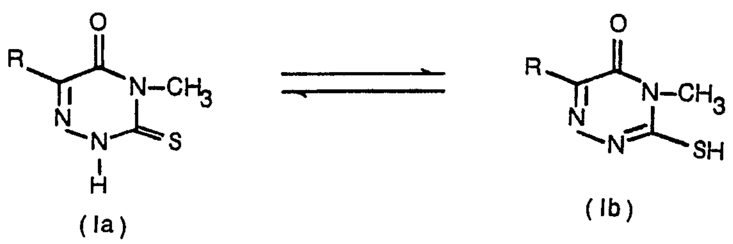

(Ia) (Ib) (I)

dans lesquelles

R représente un reste alkyle de 1 à 4 atomes de carbone, à chaîne droite ou à chaîne ramifiée, éventuellement substitué par un radical alkoxy ayant 1 à 4 atomes de carbone, carbalkoxy ayant 1 à 4 atomes de carbone dans le groupe alkoxy, nitro, cyano et/ou halogéno; un reste cycloalkyle de 3 à 6 atomes de carbone dans le noyau, éventuellement substitué par un radical alkyle, alkoxy ou carbalkoxy ayant chacun jusqu'à 4 atomes de carbone, nitro, cyano et/ou halogéno; un reste phényle ou naphtyle éventuellement substitué par un radical alkyle, alkoxy ou carbalkoxy ayant chacun jusqu'à 4 atomes de carbone, nitro et/ou halogéne; ou un reste hétérocyclique pentagonal ou hexagonal éventuellement substitué par un radical alkyle, alkoxy ou carbalkoxy ayant chacun jusqu'à 4 atomes de carbone, nitro, cyano et/ou halogèno, qui peut contenir dans le noyau 1 à 3 hétéroatomes tels que des atomes d'oxygène, de soufre et/ou d'azote et qui peut en outre être condensé avec un noyau benzénique, en faisant réagir des cyanures d'acyle de formule générale II

$$R—CO—CN \qquad (II)$$

dans laquelle

R a la définition indiquée ci-dessus,
avec un anhydride d'acide carboxylique de formule générale III

$$R^1—CO—O—CO—R^1 \quad (III)$$

dans laquelle
$R^1$ représente un reste alkyle de 1 à 4 atomes de carbone éventuellement substitué par du chlore ou un reste phényle,
en présence d'un acide fort, choisi dans le groupe comprenant des acides inorganiques, des acides de Lewis, des acides sulfoniques et phosphoniques aliphatiques ou aromatiques ainsi que des acides halogénalcanecarboxyliques, et le cas échéant en présence d'un solvant à des températures comprises entre −50 et +150°C, puis on fait réagir le mélange réactionnel ainsi obtenu directement avec le 4-méthylthiosemicarbazide ($CH_3$—NH—CS—NH—$NH_2$).

2. Procédé suivant la revendication 1, caractérisé en ce qu'on conduit la réaction à des températures comprises entre 0 et 100°C.

3. Procédé suivant la revendication 1, caractérisé en ce qu'on utilise le cyanure d'acyle (II) et l'anhydride d'acide carboxylique (III) dans le rapport molaire de 1:0,5—6.

4. Procédé suivant la revendication 3, caractérisé en ce qu'on utilise le cyanure d'acyle (II) et l'anhydride d'acide carboxylique (III) dans un rapport molaire de 1:0,8—4.

5. Procédé suivant la revendication 4, caractérisé en ce qu'on utilise le cyanure d'acyle (II) et l'anhydride d'acide carboxylique (III) dans un rapport molaire de 1:1—2.

6. Procédé suivant la revendication 1, caractérisé en ce qu'on utilise le cyanure d'acyle (II) et l'acide fort dans un rapport molaire de 1:0,5—10.

7. Procédé suivant la revendication 6, caractérisé en ce qu'on utilise le cyanure d'acyle (II) et l'acide fort dans un rapport molaire de 1:0,8—8.

8. Procédé suivant la revendication 7, caractérisé en ce qu'on utilise le cyanure d'acyle (II) et l'acide fort dans un rapport molaire de 1:1—4.

9. Procédé suivant la revendication 1, caractérisé en ce qu'on utilise l'anhydride d'acide carboxylique (III) et l'acide fort dans un rapport molaire de 1:2.

10. Procédé suivant la revendication 1, caractérisé en ce qu'on utilise le cyanure d'acyle (II), l'anhydride d'acide carboxylique (III), l'acide fort et le 4-méthylthiosemicarbazide dans la proportion molaire de 1:1:2:1 à 1:2:4:1.

11. Procédé suivant la revendication 1, caractérisé en ce qu'on utilise le cyanure de pivaloyle comme cyanure d'acyle de formule (II).

12. Procédé suivant la revendication 1, caractérisé en ce qu'on utilise l'anhydride d'acide acétique comme anhydride d'acide carboxylique de formule (III).

13. Procédé suivant la revendication 1, caractérisé en ce qu'on utilise l'acide sulfurique concentré comme acide fort.

## Claims

1. Process for the preparation of 4-methyl-5-oxo-3-thioxo-tetrahydro-1,2,4-(2H, 4H)-triazines (I), which can occur in the two tautomeric forms (Ia) and (Ib)

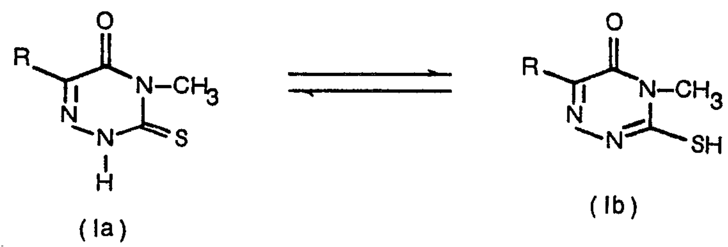

(I)

wherein
R represents a straight-chain or branched alkyl radical which has 1 to 4 carbon atoms and is optionally substituted by alkoxy having 1 to 4 carbon atoms, carbalkoxy having 1 to 4 carbon atoms in the alkoxy group, nitro, cyano and/or halogen; or represents a cycloalkyl radical which has 3 to 6 carbon atoms in the ring system and is optionally substituted by alkyl, alkoxy or carbalkoxy having in each case up to 4 carbon atoms, nitro, cyano and/or halogen; or represents a phenyl or naphthyl radical which is optionally substituted by alkyl, alkoxy or carbalkoxy having in each case up to 4 carbon atoms, nitro and/or halogen; or represents a 5-membered or 6-membered heterocyclic radical which can contain 1 to 3 hetero atoms such as oxygen, sulphur and/or nitrogen in the ring and which may additionally be fused to a benzene ring and which is optionally substituted by alkyl, alkoxy, or carbalkoxy having in each case up to 4 carbon atoms, nitro, cyano and/or halogen,
characterized in that acyl cyanides of the general formula II

$$R—CO—CN \quad (II)$$

$$R^1—CO—O—CO—R^1 \quad (III)$$

in which

$R^1$ represents an optionally chlorine-substituted alkyl radical having 1 to 4 carbon atoms or a phenyl radical,

in the presence of a strong acid, selected from the group comprising inorganic acids, Lewis acids, aliphatic or aromatic sulphonic and phosphonic acids and halogenalkane carboxylic acids, and, if appropriate, in the presence of a solvent, at temperatures between −50 and +150°C, and the reaction mixture thus obtained is then reacted directly with 4-methyl-thiosemicarbazide ($CH_3$—NH—CS—NH—$NH_2$).

2. Process according to Claim 1, characterized in that the reaction is carried out at temperatures between 0 and 100°C.

3. Process according to Claim 1, characterized in that the acyl cyanide (II) and the carboxylic acid anhydride (III) are employed in a molar ratio of 1:0.5—6.

4. Process according to Claim 3, characterized in that the acyl cyanide (II) and the carboxylic acid anhydride (III) are employed in a molar ratio of 1:0.8—4.

5. Process according to Claim 4, characterized in that the acyl cyanide (II) and the carboxylic acid anhydride (III) are employed in a molar ratio of 1:1—2.

6. Process according to Claim 1, characterized in that the acyl cyanide (II) and the strong acid are employed in a molar ratio of 1:0.5—10.

7. Process according to Claim 6, characterized in that the acyl cyanide (II) and the strong acid are employed in a molar ratio of 1:0.8—8.

8. Process according to Claim 7, characterized in that the acyl cyanide (II) and the strong acid are employed in a molar ratio of 1:1—4.

9. Process according to Claim 1, characterized in that the carboxylic acid anhydride (III) and the strong acid are employed in a molar ratio of 1:2.

10. Process according to Claim 1, characterized in that the acyl cyanide (II), the carboxylic acid anhydride (III), the strong acid and 4-methyl-thiosemicarbazide are employed in a molar ratio of from 1:1:2:1 to 1:2:4:1.

11. Process according to Claim 1, characterized in that pivaloyl cyanide is employed as the acyl cyanide of the formula (II).

12. Process according to Claim 1, characterized in that acetic acid anhydride is employed as the carboxylic acid anhydride of the formula (III).

13. Process according to Claim 1, characterized in that concentrated sulphuric acid is employed as the strong acid.